# EUROPÄISCHE PATENTSCHRIFT

(11) **EP 2 049 895 B1**
(45) Veröffentlichungstag und Bekanntmachung des Hinweises auf die Patenterteilung: **29.12.2010**
(21) Anmeldenummer: 07788207.4
(22) Anmeldetag: 03.08.2007
(51) Int. Cl.: G01N 33/487, A61B 5/00

(54) **ELEKTROCHEMISCHER SENSOR ZUR ERMITTLUNG EINER ANALYT-KONZENTRATION**
ELECTROCHEMICAL SENSOR FOR THE DETERMINATION OF AN ANALYTE CONCENTRATION
CAPTEUR ELECTROCHIMIQUE POUR DETERMINER UNE CONCENTRATION D'ANALYTE

(30) Priorität: 08.08.2006 DE 102006037328; 31.10.2006 EP 06123227
(43) Veröffentlichungstag der Anmeldung: 22.04.2009
(73) Patentinhaber: F.Hoffmann-La Roche AG, 4070 Basel (CH); Roche Diagnostics GmbH, 68305 Mannheim (DE)
(72) Erfinder: MARQUANT, Michael, 68309 Mannheim (DE); BAINCZYK, Gregor, 68199 Mannheim (DE)
(74) Vertreter: Stößel, Matthias
(86) Internationale Anmeldenummer: PCT/EP2007/058074
(87) Internationale Veröffentlichungsnummer: WO 2008/017645

(56) Entgegenhaltungen:
- EP-A- 0 567 725
- DE-A1- 4 105 222
- DE-U1- 20 009 392
- US-A- 6 004 818
- US-A1- 2005 143 635

## Beschreibung

### Technisches Gebiet

Die Erfindung bezieht sich auf einen elektrochemischen Sensor zur Ermittlung einer Konzentration mindestens eines Analyten in einem Medium, insbesondere einem Körpergewebe und/oder einer Körperflüssigkeit, ferner einer Vorrichtung, an welcher der elektrochemische Sensor eingesetzt wird, eine Verwendung des elektrochemischen Sensors sowie der Vorrichtung und ein Verfahren zur Herstellung des elektrochemischen Sensors. Derartige Sensoren oder Vorrichtungen werden insbesondere im Bereich der Medizintechnik eingesetzt, beispielsweise um elektrochemisch eine Konzentration von Glucose (insbesondere Blutglucose oder Glucose in einer Gewebeflüssigkeit), Lactat oder anderen Analyten, insbesondere von Metaboliten, zu bestimmen.

### Stand der Technik

Die Bestimmung der Blutglucosekonzentration sowie eine entsprechende Medikation ist für Diabetiker ein essenzieller Bestandteil des Tagesablaufes. Dabei ist die Blutglucosekonzentration schnell und einfach mehrmals am Tage, typischerweise 2- bis 7-mal zu bestimmen, um gegebenenfalls entsprechende medizinische Maßnahmen ergreifen zu können. In vielen Fällen erfolgt dabei eine Modifikation mittels automatischer Systeme, insbesondere mit Insulinpumpen.

Um den Tagesablauf des Diabetikers nicht mehr als unbedingt nötig einzuschränken, werden häufig entsprechend mobile Geräte eingesetzt, welche einfach zu transportieren und zu handhaben sein sollten, so dass eine Messung der Blutglucosekonzentration, beispielsweise am Arbeitsplatz oder in der Freizeit, problemlos erfolgen kann.

Derzeit sind verschiedene mobile Geräte erhältlich, welche teilweise nach unterschiedlichen Messmethoden und unter Verwendung unterschiedlicher Diagnoseverfahren funktionieren. Ein erstes Messverfahren beruht beispielsweise auf einer elektrochemischen Messmethode, wobei eine Blutprobe, welche vom Patienten durch Perforieren einer Hautschicht mittels einer Lanzette dem Körpergewebe entnommen wird, auf eine mit Enzymen und Mediatoren beschichtete Elektrode appliziert wird. Entsprechende Teststreifen für derartige elektrochemische Messverfahren sind beispielsweise in US 5,286,362 beschrieben. Andere bekannte Messmethoden verwenden optische Messverfahren, welche beispielsweise darauf beruhen, dass die zu detektierende Substanz (Analyt) mit bestimmten Nachweisreagenzien reagieren kann, wobei eine Farbänderung des Reaktionsgemisches eintritt. Systeme zum Nachweis derartiger Farbreaktionen und somit zum Nachweis der entsprechenden Analyten sind aus CA 2,050,677 bekannt.

Die beschriebenen Nachweisverfahren beruhen überwiegend darauf, dass ein Patient zunächst eine entsprechende Probe der zu untersuchenden Körperflüssigkeit entnimmt, wobei es sich sowohl um eine Blutprobe als auch um eine Urinprobe handeln kann und diese dann entsprechend mittels der Testvorrichtung untersucht. Dieses Verfahren beinhaltet jedoch verschiedene Nachteile. So ist dieses Verfahren zum einen äußerst aufwändig und setzt mehrere Handhabungsschritte voraus. So muss beispielsweise eine Lanzette bereitgestellt und gespannt werden, anschließend mittels dieser Lanzette eine Hautschicht perforiert werden, dann ein so erzeugter Blutstropfen auf einen Teststreifen aufgetragen werden und anschließend dieser Teststreifen mittels eines entsprechenden Gerätes ausgewertet werden. Für viele Patienten, insbesondere ältere Menschen und Kinder, sind diese Handhabungsschritte oftmals nur schwer durchzuführen, da die Patienten beispielsweise in ihrer motorischen Fähigkeit und ihrem Sehvermögen eingeschränkt sind. Weiterhin lassen sich diese Verfahrensschritte nur in wenigen Fällen diskret durchführen, so dass beispielsweise ein Schutz der Privatsphäre des Patienten bei einer Messung am Arbeitsplatz in nur unzureichendem Maße gewahrt ist. Auch kann eine Fehlbedienung im Rahmen des Messverfahrens leicht zu falschen Messwerten führten, mit teilweise fatalen Folgen einer auf falschen Messergebnissen aufbauenden falschen Medikation.

Aus dem Stand der Technik sind daher Systeme bekannt, welche kontinuierlich Messdaten generieren und welche alternativ oder zusätzlich zu den oben beschriebenen Systemen oder Verfahren eingesetzt werden können, z.B. um die Zahl der Einzelmessvorgänge zu reduzieren. So sind beispielsweise Systeme kommerziell erhältlich, welche einen Membran-Schlauch im Unterhautgewebe umfassen, durch den eine Transportflüssigkeit gepumpt wird. Über die Membran diffundiert Glucose in die Transportflüssigkeit, welche dann wiederum zu einer elektrochemischen Messzelle befördert wird. In der elektrochemischen Messzelle wird dann die Glucosekonzentration gemessen. Nachteilig an einer derartigen Anordnung zur kontinuierlichen Messwerterzeugung ist jedoch, dass hierbei vom Patienten ständig ein Vorrat an Transportflüssigkeit sowie ein entsprechender Abfallbehälter zur Aufnahme kontaminierter Transportflüssigkeit mitgeführt werden muss.

Weitere aus dem Stand der Technik bekannte Sensortypen zur kontinuierlichen Messwerterzeugung sind ausgestaltet, um in ein Körpergewebe implantiert zu werden. So offenbart beispielsweise US 6,892,085 B2 ein gekapseltes Glucosesensorsystem, welches einen Glucosesensor und eine Schutzkapsel umfasst. Dabei sind drei Elektroden, eine Arbeitselektrode, eine Gegenelektrode und eine Referenzelektrode vorgesehen, welche auf einer Seite eines Substrats aufgebracht sind. Zur besseren Implantierbarkeit ist diese Elektrodenanordnung in einer Hohlnadel integriert, welche in Körpergewebe eingestochen wird. Nach dem Einführen werden die Hohlnadeln, welche lediglich als Einführhilfen dienen, wieder aus dem Gewebe herausgezogen, und nur die Sensoren verbleiben im Körpergewebe. Auch US 5,591,139 offenbart ein implantierbares Mikronadelsystem, mittels dessen für Diagnosezwecke Substanzen aus lebendem Gewebe entnommen werden können. Dabei wird ein geätztes dreidimensionales Substrat eingesetzt.

Ein Hauptvorteil der kontinuierlich messenden Systeme besteht darin, dass auch kürzere periodische Schwankungen der Glucosekonzentration (zeitliche Verläufe) im Zusammenhang mit der Nahrungsaufnahme und körperlicher Betätigung erfasst werden können. Dies ist für das "Einstellen" eines Diabetikers von großer Bedeutung.

Die aus dem Stand der Technik bekannten implantierbaren Sensoren sind bezüglich ihres Aufbaus und ihrer Herstellung jedoch äußerst aufwändig. Wird vorausgesetzt, dass es sich bei diesen Sensoren um nur für kurze Zeit (typischerweise etwa eine Woche) verwendbare Einwegsensoren handelt, so wird deutlich, dass die bei den aus dem Stand der Technik bekannten Sensoren eingesetzten Verfahren derartigen Anforderungen an Einwegartikeln nicht gerecht werden. So ist beispielsweise für die Herstellung des aus US 5,591,139 bekannten Sensors ein aufwändiges Mikrostrukturierungsverfahren erforderlich, insbesondere ein lithographisches Verfahren. Derartige Verfahren sind jedoch mit der Herstellung kostengünstiger Einwegartikel nicht vereinbar. Auch zur Herstellung des aus US 6,892,085 B2 bekannten Sensors sind aufwändige Strukturierungsverfahren erforderlich, da die Elektrodenpads sorgfältig strukturiert werden müssen. Angesichts der geringen Größe dieser Elektroden sind dafür ebenfalls lithographische Verfahren erforderlich, was die Kosten zur Herstellung derartiger Sensoren wiederum in die Höhe treibt.

Auch sind lithographische Verfahren, insbesondere das mit diesen Verfahren verbundene Ätzen von Metallschichten, nicht immer so zuverlässig, wie dies für die Herstellung medizintechnischer Produkte erforderlich ist. Insbesondere kann es vereinzelt vorkommen, dass einzelne Elektroden noch durch "Brücken" oder Stege miteinander verbunden sind, so dass die Funktionalität der Sensoren aufgrund von Herstellungsproblemen leicht beeinträchtigt oder sogar ganz verhindert sein kann. Ein weiterer Nachteil der aus dem Stand der Technik bekannten Sensoren, wie sie beispielsweise aus der US 6,892,085 B2 und US 5,591,139 hervorgehen, besteht in der Verwendung einer Hohlnadel bzw. in der Verwendung einer Kapillare.

Anstelle der vorhergehend beschriebenen implantierbaren Sensoren, bei denen zur Strukturierung der Elektrodenpads Mikrostrukturierungsverfahren, so zum Beispiel ein lithographisches Verfahren, eingesetzt werden, können implantierbare Sensoren, wie zum Beispiel aus WO 90/10861 bekannt, drahtförmig ausgebildet werden. Aus WO 90/10861 A1 geht ein Sensorsystem hervor, dessen einzelne Drähte in eine isolierende Masse eingebettet sind. Die aktiven Messflächen sind jeweils die Stirnseiten der Drähte innerhalb einer Ebene, die durch einen Trennvorgang oder Ähnliches freigelegt wird. Das Sensorsystem gemäß WO 90/10861 A1 ist mehrfach verwendbar und wird in ein entsprechendes Messgerät eingesetzt. Die Probe wird innerhalb des Messgerätes auf die zuvor freigelegten Stirnseiten der Drähte aufgebracht (in vitro-Messung).

Aus US 4,805,624 geht eine gestreckt ausgebildete drahtförmige Arbeitselektrode eines elektrochemischen Sensors hervor, die in einem Glasstab aufgenommen ist. Neben der Arbeitselektrode umfasst der aus US 4,805,624 bekannte elektrochemische Sensor eine Referenzelektrode sowie eine Gegenelektrode, die jedoch um die vom Glasstab umschlossene Arbeitselektrode spiralförmig gewickelt sind. Dieser elektrochemische Sensor umfasst einen speziellen Elektrolyten, der nicht durch die Körperflüssigkeit dargestellt wird. Nachzuweisende Glucose diffundiert über eine Membran, die einen Hohlzylinder, in dem der Elektrolyt aufgenommen ist, abschließt, in das Innere einer Messzelle.

Die vorstehend diskutierten Lösungen gemäß dem Stand der Technik sind nur in einem sehr eingeschränkten Bereich mit einem Körpergewebe in Kontakt. Die Elektrodenanordnung, üblicherweise eine Arbeitselektrode, eine Gegenelektrode und eine Referenzelektrode umfassend, ist lokal sehr eingeschränkt, d.h. vermag nur aussagefähige Ergebnisse in einem sehr kleinen Bereich des Körpergewebes zu erfassen. Die Funktion der aus dem Stand der Technik bekannten, auch implantierbaren, Sensoren kann durch lokale Gewebeinhomogenitäten, wie zum Beispiel Wundeffekte oder Fetteinlagerungen, gestört werden. Ferner wirken sich Sensormembraneigenschaften, insbesondere gemäß US 4,805,624, negativ auf die Messwerte aus. Die aus US 6,892,085 B2 sowie US 5,591,139 bekannten Elektrodenpads weisen in einer Ebene nebeneinander liegende Elektroden auf, bei denen abhängig von den gewählten Mikrostrukturierungsverfahren die notwendige Miniaturisierbarkeit erheblich eingeschränkt ist. Der Nachteil der aus dem Stand der Technik bekannten, auch implantierbaren Sensoren ist darin zu erblicken, dass zu einer Großserienfertigung erforderliche kostengünstige Herstellverfahren, die im Rahmen einer Massenproduktion eingesetzt werden könnten, nicht genutzt werden können.

Ein implantierbarer Sensor mit helixartig um einen Stab gewickelten Elektroden wird in US2005/0143635 beschrieben.

### Aufgabe der Erfindung

Die Aufgabe der Erfindung liegt darin, einen Sensor zur Verfügung zu stellen, welcher einfach und kostengünstig mittels eines zuverlässigen Herstellungsverfahrens herstellbar ist und welcher die Nachteile der aus dem Stand der Technik bekannten Sensoren und Verfahren vermeidet.

### Darstellung der Erfindung

Die Aufgabe wird durch die Erfindung mit den Merkmalen der unabhängigen Ansprüche gelöst. Vorteilhafte Weiterbildungen der Erfindung sind den Unteransprüchen zu entnehmen.

Es wird ein elektrochemischer Sensor zur Ermittlung einer Konzentration mindestens eines Analyten in einem Medium, insbesondere einem Körpergewebe und/oder einer Körperflüssigkeit, vorgeschlagen. Der elektrochemische Sensor weist ein Isolationselement und mindestens zwei Elektroden auf, wobei die mindestens zwei Elektroden mindestens eine Arbeitselektrode und mindestens eine weitere Elektrode, insbesondere mindestens eine Gegenelektrode und/oder mindestens eine Referenzelektrode, umfassen. Die mindestens zwei Elektroden sind zumindest teilweise als im Wesentlichen parallel zueinander verlaufende drahtförmige Elektroden mit umfangsseitigen Elektrodenflächen ausgebildet. Die mindestens zwei Elektroden erstrecken sich entlang eines profilförmig ausgeführten Isolationselements. Das profilförmig ausgeführte Isolationselement weist mindestens zwei Aufnahmefächer für die mindestens zwei Elektroden auf.

Vorzugsweise ist der elektrochemische Sensor derart ausgestaltet, dass dieser in ein Körpergewebe implantiert und/oder subkutan eingeführt werden kann. Zu diesem Zweck ist daher zumindest die freiliegende Sensoroberfläche vorzugsweise biokompatibel ausgestaltet, so dass insbesondere keine Zellgifte in das Körpergewebe diffundieren können bzw. in Kontakt mit dem Körpergewebe gelangen.

Bei dem Analyten kann es sich zum Beispiel um Glucose, Lactat, Hormone oder andere Analyten handeln, welche insbesondere in der Medizin eine Rolle spielen. Alternativ oder zusätzlich kann der elektrochemische Sensor jedoch auch zur Messung anderer Arten von

Analyten eingesetzt werden.

Ein Grundgedanke der Erfindung besteht darin, den elektrochemischen Sensor so auszugestalten, dass eine Anordnung von mindestens zwei dünnen Drähten eine elektrochemische Messzelle bildet. Die Anordnung von dünnen Drähten stellt gleichzeitig die elektrische Verbindung zu einer geeigneten Messelektronik her. Die Messung der Analytkonzentration erfolgt dann durch elektrochemische (beispielsweise amperometrische) Messmethoden zwischen den mindestens zwei Elektroden, einer Arbeitselektrode und einer Gegenelektrode, insbesondere mittels einer Gleichspannung. Eine Referenzelektrode zur stromlosen Messung des Arbeitselektrodenpotenzials kann zusätzlich eingesetzt werden.

Um eine möglichst kompakte Bauweise des elektrochemischen Sensors zu erreichen, werden die Einzelelektroden der Elektrodenanordnung zumindest in einem Abschnitt im Wesentlichen parallel, vorzugsweise exakt parallel (d. h. eine Winkelabweichung von der Parallelen um vorzugsweise nicht mehr als 5°, besonders bevorzugt von nicht mehr als 1 °), zueinander ausgerichtet und bevorzugt durch ein Isolationsprofil gegeneinander isoliert. Auf diese Weise lassen sich günstigere Eigenschaften des elektrochemischen Sensors erreichen, da der Sensor entlang seiner Längserstreckung eine gute Homogenität aufweist. Insbesondere weist die Zellenbreite des Sensors (d. h. Dicke der Schichten, Elektrodenabstand etc.) eine hohe Gleichförmigkeit und geringe Toleranzen auf.

Die Elektrodenanordnung umfasst mindestens zwei Elektroden, wobei es sich um mindestens eine Arbeitselektrode und mindestens eine weitere Elektrode handelt, wobei die mindestens eine weitere Elektrode insbesondere mindestens eine Gegenelektrode und/oder mindestens eine Referenzelektrode umfassen sollte. Bei dem erfindungsgemäß vorgeschlagenen elektrochemischen Sensor sind die Arbeitselektrode und die mindestens eine weitere Elektrode durch das Isolationsprofil voneinander getrennt. Bei dem erfindungsgemäß vorgeschlagenen elektrochemischen Sensor ist das Isolationsprofil des elektrochemischen Sensors durch ein elektrisch nicht leitendes Material, wie zum Beispiel ein Kunststoffmaterial, dargestellt.

Die Herstellung der Elektrodenanordnung für den erfindungsgemäß vorgeschlagenen elektrochemischen Sensor kann unter Einsatz von effektiven, kostengünstigen Herstellungsverfahren erfolgen. In einem ersten Herstellungsschritt wird die mindestens eine Arbeitselektrode der erfindungsgemäß vorgeschlagenen Elektrodenanordnung, die bevorzugt aus einem für elektrochemische Zwecke geeignetem Material, wie zum Beispiel Gold, Palladium, Platin, und/oder Kohlenstoff gefertigt ist, mit einem zum Nachweis des Analyten geeigneten Reagenz beschichtet. Dies kann innerhalb einer Ringdüsenbeschichtung erfolgen, wobei die im Rahmen der Ringdüsenbeschichtung eingesetzte Ringdüse, die einen kreisförmigen Querschnitt aufweisende, mindestens eine Arbeitselektrode ringförmig umschließt und eine Beschichtung der gesamten Mantelfläche der mindestens einen Arbeitselektrode in einem Arbeitsgang durchgeführt werden kann. Innerhalb dieses Herstellungsschrittes bildet die mindestens eine Arbeitselektrode einen langen endlosen Draht, der in vorteilhafter Weise im Rahmen einer Ringdüsenbeschichtung allseitig und in gleichmäßiger Film- oder Beschichtungsdicke beschichtet werden kann. Nach erfolgter Ringdüsenbeschichtung der mindestens einen Arbeitselektrode durchläuft die beschichtete mindestens eine Arbeitselektrode eine Trocknungsstation, welche bevorzugt als Höhlzylinder beschaffen ist, so dass die die Trocknungsstation durchlaufende beschichtete mindestens eine Arbeitselektrode gleichmäßig getrocknet wird.

In einem nachfolgenden Verfahrensschritt werden die mindestens eine, nunmehr beschichtete Arbeitselektrode, gegebenenfalls die mindestens eine Referenzelektrode und des Weiteren die mindestens eine Gegenelektrode, die unbeschichtet bleiben kann und aus einem für elektrochemische Zwecke geeignetem Material, wie zum Beispiel Gold, Silber, Palladium, Platin oder Kohlenstoff, gefertigt sein kann, zusammengeführt. Neben den genannten drei Elektroden der Elektrodenanordnung wird im Rahmen der Zusammenführung der drei genannten Elektroden auch das Isolationsprofil erarbeitet. Bei dem Isolationsprofil, welches zum Beispiel eine Y- oder Sterngeometrie aufweisen kann, handelt es sich bevorzugt um ein aus Kunststoff gefertigtes Strangextrusionsprofil, welches beispielsweise durch Mikroextrusion hergestellt wird. Wird beispielsweise ein Strangextrusionsprofil mit Y-Geometrie eingesetzt, so entstehen in vorteilhafter Weise drei Aufnahmetaschen, in welche die mindestens eine Arbeitselektrode, die mindestens eine Gegenelektrode sowie die mindestens eine Referenzelektrode eingelassen werden können.

Nach der Zusammenführung des Strangextrusionsprofils, d.h. des Isolationsprofils, und der mindestens einen Arbeitselektrode, der mindestens einen Gegenelektrode sowie der mindestens eine Referenzelektrode wird eine Elektrodenanordnung in Paketform erhalten. Diese Elektrodenanordnung in Paketform kann in einem nachfolgenden Herstellungsschritt durch eine Immobilisierungsmedium-Beschichtung zur Immobilisierung der reaktiven Bestandteile im Rahmen eines bevorzugt als Ringdüsen-Beschichtungsverfahren ausgebildeten Weiterverarbeitungsvorgangs zugeführt werden. Das Aufbringen der Immobilisierungsmedium-Beschichtung zur Immobilisierung reaktiver Bestandteile, was bevorzugt mit einem Ringdüsen-Beschichtungsverfahren zur Aufbringung der Immobilisierungsmedium-Beschichtung in einem Arbeitsgang am gesamten Umfang gemäß dem im vorhergehenden Arbeitsschritt erhaltenen Elektrodenpaket vorgenommen werden kann, kann alternativ auch an den einzelnen Elektroden, d.h. der mindestens einen Arbeitselektrode, der mindestens einen Gegenelektrode sowie der mindestens einen Referenzelektrode ausgeführt werden.

Nach dem Membran-Beschichtungsverfahren zur Aufbringung einer Membran zur Immobilisierung der reaktiven Bestandteile wird die erhaltene Elektrodenanordnung in Paketform einer Konfektionierung zugeführt. Unter Konfektionierung wird nachfolgend das Auftrennen des in Endlosform vorliegenden, mit einer Immobilisierungsmedium-Beschichtung versehenen Elektrodenpaketes verstanden, wobei das Elektrodenpaket das Isolationsprofil aufweist, welches die mindestens eine Arbeitselektrode, die mindestens eine Gegenelektrode sowie die mindestens eine Referenzelektrode voneinander isoliert. Die Konfektionierung dieser in Endlosform vorliegenden Elektrodenanordnung erfolgt durch Schneiden einzelner Sektionen der Elektrodenanordnung in Paketform. Entsprechend der Konfektionierung können variable Längen abgetrennt werden, wobei ein Ende der erhaltenen Elektrodenanordnung, d.h. einer Sektion, zum Beispiel in einem geeigneten Schneidklemmstecker montiert werden kann. Das andere Ende der abgetrennten Sektion kann in einem Formschlussteil vergossen werden, welches auch andere notwendige Funktionen eines elektrochemischen Sensors, so zum Beispiel zur Insertion in das Körpergewebe übernimmt.

### Zeichnung

Anhand der Zeichnung wird die Erfindung nachstehend eingehender beschrieben.

Es zeigt:
Figur 1 einen Querschnitt durch einen Schneidklemmstecker zur elektrischen Kontaktierung der Elektrodenanordnung des erfindungsgemäßen elektrochemischen Sensors,
Figur 2 eine schematische Seitenansicht der erfindungsgemäß vorgeschlagenen Vorrichtung zur Ermittlung einer Analytkonzentration in einem Medium mit einem Einführkopf und dem Schneidklemmstecker,
Figur 3 eine Anordnung zur Beschichtung einer Einzelelektrode der Elektrodenanordnung mit zum Aufbringen eines zum Nachweis eines Analyten geeigneten Reagenzmediums mit nachgeschalteter Trocknungsstation,
Figur 4 die Zusammenführung der mindestens einen Arbeitselektrode, der mindestens einen Gegenelektrode, eines Isolationsprofils und einer Referenzelektrode zu einer Elektrodenanordnung,
Figur 4.1 einen Schnitt durch das Isolationsprofil,
Figur 5 eine Ringdüsen-Beschichtungsstation zur Aufbringung einer eine Immobilisierung reaktiver Bestandteile ermöglichenden Membran auf die Elektrodenanordnung,
Figur 6 eine Konfektionierung der mit einer Immobilisierungsmedium-Beschichtung versehenen Elektrodenanordnung,
Figur 7 eine perspektivische Draufsicht auf die erfindungsgemäß vorgeschlagene Elektrodenanordnung,
Figur 8 ein schematisches Verfahrensschaubild, und
Figur 9 einen Ausschnitt einer Querschnittsdarstellung der Oberfläche einer Arbeitselektrode.

### Ausführungsvarianten

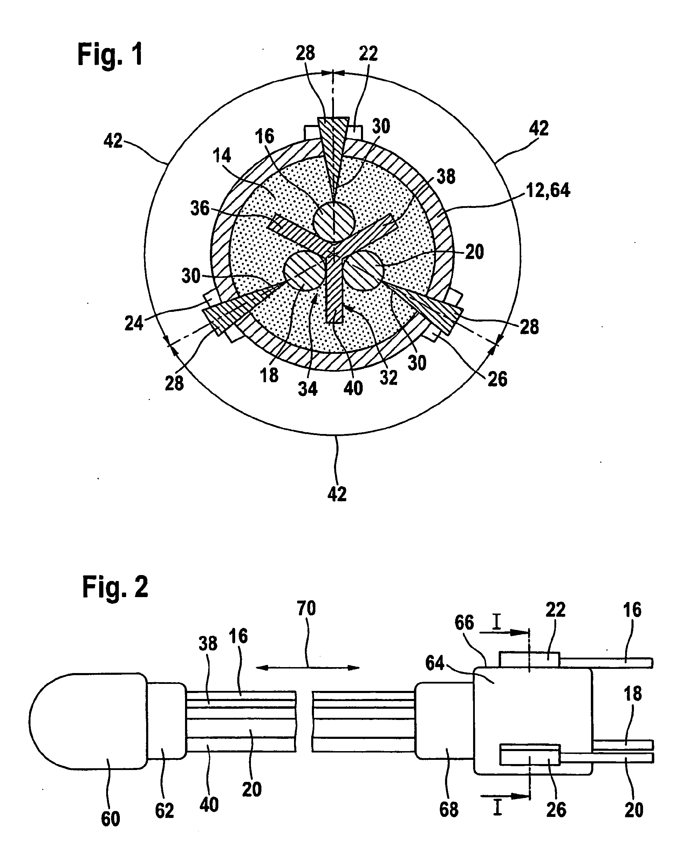

Der Darstellung gemäß Figur 1 ist ein Schnitt durch einen (in diesem Beispiel als Schneidklemmstecker ausgeführten) Stecker zu entnehmen, der eine Elektrodenanordnung für einen elektrochemischen Sensor umfasst.

Der in Figur 1 dargestellte Querschnitt durch einen elektrochemischen Sensor 10 zeigt eine Elektrodenanordnung 16, 18, 20. Die Elektrodenanordnung 16, 18, 20 ist von einem Mantel 12 umschlossen, der ein Einbettungsmaterial 14 umschließen kann. Die Elektrodenanordnung umfasst mindestens eine Arbeitselektrode 16, mindestens eine Gegenelektrode 18 und mindestens eine Referenzelektrode 20. Die Elektrodenanordnung 16, 18, 20 erstreckt sich in der Darstellung gemäß Figur 1 senkrecht in die Zeichenebene, wobei die mindestens eine Arbeitselektrode 16, die mindestens eine Gegenelektrode 18 und die mindestens eine Referenzelektrode 20 parallel zueinander verlaufen.

Die mindestens eine Arbeitselektrode 16, die mindestens eine Gegenelektrode 18 sowie die mindestens eine Referenzelektrode 20 bilden eine elektrochemische Messzelle und gleichzeitig den Körper des elektrochemischen Sensors 10. Die mindestens eine Arbeitselektrode 16 wird aus einem für elektrochemische Zwecke geeigneten Material, wie zum Beispiel Gold, Silber, Palladium, Platin oder Kohlenstoff, gefertigt. Auch die Verwendung weiterer Edelmetalle oder sonstiger Metalle oder Metalllegierungen, auch in Mehrschichtanordnung, ist denkbar. Die mindestens eine Arbeitselektrode 16 kann zum Nachweis des Analyten mit einem geeigneten Reagenz beschichtet werden, wie nachfolgend noch eingehender beschrieben wird.

Die mindestens eine Gegenelektrode 18 wird ebenfalls aus einem für elektrochemische Zwecke geeigneten Material, wie zum Beispiel Gold, Silber, Palladium, Platin oder Kohlenstoff, gefertigt und kann unbeschichtet bleiben oder auch ein- oder mehrlagig beschichtet werden. Wiederum ist hierbei alternativ oder zusätzlich, wie auch bei den anderen Elektroden, auch die Verwendung weiterer Metalle (vorzugsweise Edelmetalle) oder Metalllegierungen oder Mehrschichtmetalle möglich. Die mindestens eine Referenzelektrode 20 wird bevorzugt aus einem Silberdraht gefertigt, der an seiner Mantelfläche mit Silberchlorid belegt ist. Die mindestens eine Referenzelektrode 20 wird zur stromlosen Messung des Potenzials der mindestens einen Arbeitselektrode eingesetzt.

Aus der Darstellung gemäß Figur 1 geht darüber hinaus hervor, dass am Umfang des Mantels 12 des elektrochemischen Sensors 10 jeweils ein Anschluss 22 zum Herstellen einer elektrischen Kontaktierung der mindestens einen Arbeitselektrode 16, ein Anschluss 24 zur elektrischen Kontaktierung der mindestens einen Gegenelektrode 18 sowie ein elektrischer Anschluss 26 zur elektrischen Kontaktierung der mindestens einen Referenzelektrode 20 ausgebildet sind. In der Darstellung gemäß Figur 1 ist die der Anzahl der Elektroden 16, 18, 20 entsprechende Anzahl der Anschlüsse 22, 24, 26 in einer Umfangsteilung 42 angeordnet, die im dargestellten schematisch wiedergegebenen Ausführungsbeispiel 120° beträgt. Je nach Anzahl der an der Elektrodenanordnung 16, 18, 20 eingesetzten Einzelelektroden wird eine dazu korrespondierende Anzahl von Anschlüssen 22, 24, 26 am Mantel 12 des elektrochemischen Sensors 10 vorgesehen. Bei dem in Figur 1 dargestellten Ausführungsbeispiel werden die mindestens eine Arbeitselektrode 16, die mindestens eine Gegenelektrode 18 und die mindestens eine Referenzelektrode 20 über Kontaktierungselemente 28 elektrisch kontaktiert. Die Kontaktierungselemente 28 können als Schneidklemmen ausgebildet werden, die eine Schneide 30 umfassen. Ein spitzes Ende der an den Kontaktierungselementen 28 keilförmig ausgebildeten Schneide 30 kontaktiert jeweils den Umfang der mindestens einen Arbeitselektrode 16, der mindestens einen Gegenelektrode 18 sowie der mindestens einen Referenzelektrode 20. Alternativ zu einer Schneidklemmverbindung können auch die Enden der Drähte freigelegt werden und separat auf eine Anschlussplatine gelötet, gebondet oder geklebt sein. Anschließend kann diese Anordnung beispielsweise vergossen werden, beispielsweise in einem Steckergehäuse.

Aus der Schnittdarstellung gemäß Figur 1 geht ferner hervor, dass die mindestens eine Arbeitselektrode 16, die mindestens eine Gegenelektrode 18 sowie die mindestens eine Referenzelektrode 20 durch ein Isolationsprofil 32 gegen einander isoliert sind. In der Darstellung gemäß Figur 1 ist das Isolationsprofil 32 in Y-Geometrie ausgebildet, wodurch sich jeweils ein Aufnahmefach, das die mindestens eine Arbeitselektrode 16, die mindestens eine Gegenelektrode 18 sowie die mindestens eine Referenzelektrode 20 aufnimmt, ergibt. Bevorzugt wird das Isolationsprofil 32 als Strangextrusionsprofil gefertigt. Anstelle der in Figur 1 dargestellten Y-Geometrie 34 kann das Isolationsprofil 32 auch eine andere Konfiguration aufweisen, so zum Beispiel X-förmig oder T-förmig ausgeführt werden. Auch eine kreuzförmige Ausbildung der Geometrie des Isolationsprofils 32 ist möglich. Es sollte sichergestellt sein, dass durch die Geometrie 34 des Isolationsprofils gewährleistet ist, dass die mindestens eine Arbeitselektrode 16, die mindestens eine Gegenelektrode 18 sowie die mindestens eine Referenzelektrode 20, die parallel zueinander verlaufen, durch das Isolationsprofil, zum Beispiel durch das Isolationsprofil 32, durch Stege voneinander getrennt sind.

Das in Figur 1 im Schnitt dargestellte Isolationsprofil 32 in Y-Geometrie 34 umfasst einen ersten Steg 36, welcher die mindestens eine Gegenelektrode 18 von der mindestens einen Arbeitselektrode 16 trennt. Über einen zweiten Steg 38 des Isolationsprofils 32 erfolgt eine Trennung der mindestens einen Arbeitselektrode 16 von der mindestens einen Referenzelektrode 20, die wiederum durch einen dritten Steg 40 des lsolationsprofils 32 von der mindestens einen Gegenelektrode 18 der Elektrodenanordnung 16, 18, 20 getrennt ist.

Aus der Darstellung gemäß Figur 2 geht eine Vorrichtung, welche den elektrochemischen Sensor gemäß Figur 1 umfasst, hervor.

Der schematischen Darstellung gemäß Figur 2 ist entnehmbar, dass der elektrochemische Sensor 10 Teil einer Vorrichtung ist, welche einen Einführkopf 60 aufweist. Der Einführkopf 60 ist an seinem Ende gerundet ausgebildet und weist einen Durchmesser von vorzugsweise < 1 mm, insbesondere < 500 Mikrometern und besonders bevorzugt von < 50 Mikrometern auf. Der Einführkopf 60 dient dem subkutanen Einführen des elektrochemischen Sensors in ein Körpergewebe. Der Einführkopf 60 ist über eine erste Abdichtung 32 gegenüber der Elektrodenanordnung 16, 18, 20 abgedichtet, wobei in der Darstellung gemäß Figur 2 nur die mindestens eine Arbeitselektrode 16 sowie die mindestens eine Referenzelektrode 20 dargestellt sind. Durch den mit Bezugszeichen 70 bezeichneten Doppelpfeil ist die Bewegungsrichtung in der Vorrichtung zur Bestimmung einer Analytkonzentration in einem Körpergewebe oder in einer Körperflüssigkeit angedeutet.

Alternativ zu dem in Figur 2 dargestellten Verfahren der Einführung mittels des Einführkopfes 60 sind jedoch auch andere Vorrichtungen bzw. Vorgehensweisen denkbar, um den elektrochemischen Sensor 10 einzuführen, bei welchen kein Einführkopf 60 erforderlich ist. So kann beispielsweise der elektrochemische Sensor 10 über eine geschlitzte Hohlnadel in das Gewebe eingeführt werden, wobei anstelle eines Einführkopfes 60 lediglich eine Isolation der Stirnfläche des Sensors 10 verwendet werden kann. Alternativ oder zusätzlich kann der elektrochemische Sensor 10 auch mittels einer Einführhilfe, beispielsweise einer Nadel oder einer Klinge, unter die Haut gezogen werden. In diesem Fall kann beispielsweise ein Formschlussteil eingesetzt werden, welches als Mitnehmer dient und beim Herausziehen der Einführhilfe den elektrochemischen Sensor 10 nicht wieder mit hinauszieht. Der elektrochemische Sensor 10 selbst sollte allerdings derart ausgestaltet sein, dass dieser nach Ende seiner Verwendung auch wieder relativ leicht aus dem Gewebe entfernbar ist.

Die in Figur 2 dargestellte Vorrichtung umfasst neben dem Einführkopf 60 und der Elektrodenanordnung 16, 18, 20 einen in Figur 1 im Querschnitt dargestellten Anschlussträger 64, der, wie oben stehend im Zusammenhang mit Figur 1 beschrieben, bevorzugt als Schneidklemmstecker ausgebildet ist. Der Anschlussträger 64 stellt die elektrische Kontaktierung der mindestens einen Arbeitselektrode 16, der mindestens einen Gegenelektrode 18 und der mindestens einen Referenzelektrode 20 sicher. Der Anschlussträger 64 weist einen Umfang 66 auf, an dem die in Figur 1 dargestellten Anschlüsse 22, 24, 26 zur elektrischen Kontaktierung der mindestens einen Arbeitselektrode 16, der mindestens einen Gegenelektrode 18 und der mindestens einen Referenzelektrode 20 elektrisch kontaktiert sind. Wie im Zusammenhang mit Figur 1 bereits erwähnt, werden die elektrischen Kontaktierungselemente 28 bevorzugt als Schneiden 30 umfassende keilförmige Elemente ausgebildet, welche jeweils die Umfangsfläche der mindestens einen Arbeitselektrode 16, der mindestens einen Gegenelektrode 18 sowie der mindestens einen Referenzelektrode 20 elektrische kontaktieren. Die Vorrichtung gemäß der Darstellung in Figur 2 umfasst darüber hinaus eine Auswerteeinheit, welche die Signale, die über die mindestens eine Arbeitselektrode 16 und die mindestens eine Gegenelektrode 18 übermittelt werden, auswertet und eine Bestimmung der Analytkonzentration im Körpergewebe oder in einer Körperflüssigkeit vornimmt und dem Anwender direkt anzeigt.

Der Vollständigkeit halber sei erwähnt, dass der Schnittverlauf I-I dem in Figur 1 dargestellten Schnitt durch den elektrochemischen Sensor 10 entspricht. Zwischen dem Anschlussträger 64, der bevorzugt als Formschlussteil ausgeführt werden kann, und der Elektrodenanordnung 16, 18, 20 befindet sich eine zweite Abdichtung 68. Je nach Konfektionierung der Elektrodenanordnung 16, 18, 20, mindestens eine Arbeitselektrode 16, mindestens eine Gegenelektrode 18 und mindestens eine Referenzelektrode 20 umfassend, können unterschiedliche Längen der Elektrodenanordnung 16, 18, 20 zwischen der ersten Abdichtung 62 zum Einführkopf 60 und der zweiten Abdichtung 68 zum Anschlussträger 64 realisiert werden. Entsprechend der Konfektionierung der Länge der Elektrodenanordnung 16, 18, 20 kann ein mehr oder weniger tiefes subkutanes Einschieben des elektrochemischen Sensors in ein Körpergewebe zur Bestimmung einer Analytkonzentration vorgenommen werden. Dabei bilden der erfindungsgemäßen Lösung folgend die mindestens eine Arbeitselektrode 16, die mindestens eine Arbeitselektrode 18 und die mindestens eine Referenzelektrode 20 die elektrochemische Messzelle, die nach Einführen des Einführkopfes 60 vom Körpergewebe umschlossen ist und eine Erfassung eines Analyten in einem Körpergewebe oder einer Körperflüssigkeit ermöglicht. Die Elektrodenanordnung 16, 18, 20 weist aufgrund der Ausbildung der mindestens einen Arbeitselektrode 16, der mindestens einen Gegenelektrode 18 sowie der mindestens einen Referenzelektrode 20 als drahtförmige Bauteile, eine sehr langgestreckte Oberfläche auf. Somit erfolgt aufgrund der hohen, langgestreckten Elektrodenoberfläche der elektrochemische Nachweis in einem großen Gewebebereich. Dadurch haben lokale Inhomogenitäten (z.B. isolierende Fettzellen) einen geringeren Einfluss auf den Gesamtstrom, da über einen größeren Bereich integriert wird. Ein hoher Stromumsatz ist typischerweise auch mit einem hohen Glucoseverbrauch verbunden. Dadurch kann es im Gewebe zu Verarmungen und damit zu fälschlicherweise als zu niedrig ermittelten Messwerten kommen. Ziel ist es somit, die Elektrodenfläche nicht zu wählen, jedoch gleichzeitig viel Raum im Gewebe zu erfassen. Dieses Ziel wird insbesondere durch lange, dünne Drähte erreicht. Alternativ oder zusätzlich kann der Glucoseverbrauch auch durch eine dickere Immobilisierungsschicht gedrosselt werden. Je dünner Elektrodenpaket ausgeführt ist, desto geringer sind in der Regel störende Wechselwirkungen mit dem Körpergewebe (z.B. Zellaufwachsen oder Wundheilung).

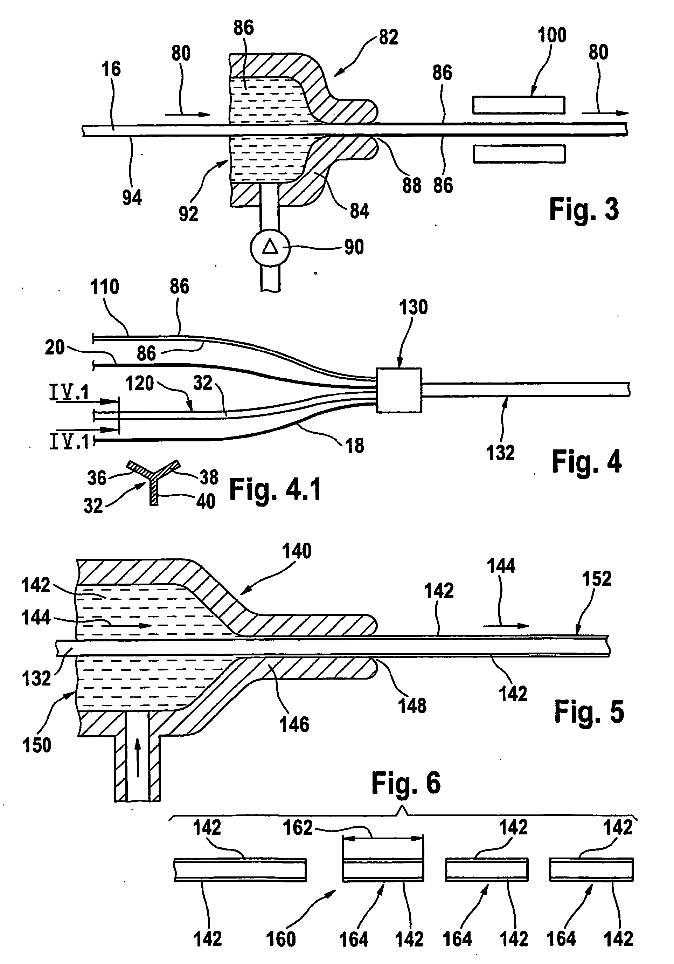

Der Darstellung gemäß Figur 3 ist ein erster Herstellungsschritt der Elektrodenkonfiguration, wie in den Figuren 1 und 2 anhand eines elektrochemischen Sensors dargestellt, zu entnehmen.

Der erfindungsgemäß vorgeschlagene elektrochemische Sensor 10 zeichnet sich durch ein Herstellungsverfahren aus, welches eine Nutzung von für eine Großserienproduktion vorteilhaften einzelnen Herstellungsschritten ermöglicht. Die Komponenten des elektrochemischen Sensors 10 sind im Wesentlichen die mindestens eine Arbeitselektrode 16, die mindestens eine Gegenelektrode 18 und die mindestens eine Referenzelektrode 20 sowie das Isolationsprofil 32. Die zur Herstellung einer ein- oder mehrlagigen Beschichtung der mindestens einen Arbeitselektrode 16, der mindestens einen Gegenelektrode 18 und der mindestens einen Referenzelektrode 20 werden nachfolgend detaillierter beschrieben.

Die mindestens eine Arbeitselektrode 16 der Elektrodenanordnung 16, 18, 20 ist zum Nachweis desjenigen Analyten in einem Körpergewebe, der zu bestimmen ist, mit einer dazu geeigneten Reagenz beschichtet. Dieser Beschichtungsschritt wird gemäß der Darstellung in Figur 3 im Rahmen einer Ringdüsen-Beschichtung 82 vorgenommen. Dazu bewegt sich die mindestens eine Arbeitselektrode 16 in Förderrichtung 80 durch eine Ringdüse 84. Die Ringdüse 84 umfasst einen Hohlraum 92, der mit einem Reagenzmedium 86 befüllt ist. Die Förderung des Reagenzmediums 86 in den Hohlraum 92 der Ringdüse 84 erfolgt durch eine Förderpumpe 90. Dadurch, dass der Hohlraum 92 der Ringdüse 84 mit dem Reagenzmedium 86 beaufschlagt ist, wird die mindestens eine in drahtförmiger Gestalt vorliegende Arbeitselektrode 16 bei der Passage des Hohlraums 92 der Ringdüse 84 in Förderrichtung 80 an ihrer Oberfläche 94 in einem Arbeitsgang mit dem Reagenzmedium 86 beschichtet. Bei dem Reagenzmedium 86 kann es sich beispielsweise um eine Mischung aus Mangandioxid (Braunstein), Graphit und GOD (Glucoseoxidase) handeln, welche Glucose katalytisch in Gluconolacton umwandeln. Die die Ringdüse 84 in Förderrichtung 80 verlassende, mindestens eine Arbeitselektrode 16 weist nach Passage einer Austrittsöffnung 88 der Ringdüse 84 eine beschichtete Oberfläche 94 auf, die durch das Reagenzmedium 86 gebildet ist. In einer der Ringdüse 84 in Förderrichtung 80 der mindestens einen Arbeitselektrode 16 nachgeschalteten Trockenstation 100 wird der Film des Reagenzmediums 86 auf der Oberfläche 94 der mindestens einen Arbeitselektrode 16 getrocknet, bevor sich die nachstehend beschriebenen Herstellungsschritte anschließen.

Figur 4 zeigt die Zusammenführung der mindestens einen beschichteten Arbeitselektrode mit der mindestens einen Gegenelektrode, der mindestens einen Referenzelektrode sowie des in Strangform vorliegenden Isolationsprofils.

Die im Rahmen des Beschichtungsschrittes 82 gemäß der Darstellung in Figur 3 mit einem Reagenzmedium 86 beschichtete mindestens eine Arbeitselektrode 16 wird als mindestens eine beschichtete Arbeitselektrode 110 einer Zusammenführungsstation 130 zugeführt. Ferner werden der Zusammenführungsstation 130 die mindestens eine, bevorzugt unbeschichtete Gegenelektrode 18 sowie die mindestens eine Referenzelektrode 20 zugeführt. Des Weiteren wird der Zusammenführungsstation 130 das bevorzugt als Strangextrusionsprofil gefertigte Isolationsprofil 32 zugeführt, welches die in Figur 4.1 dargestellte Y-Geometrie 34 aufweist. In der Zusammenführungsstation 130 werden die mindestens nunmehr beschichtete Arbeitselektrode 16, die mindestens eine, beispielsweise unbeschichtete oder auch beschichtete, Gegenelektrode 18 sowie die mindestens eine Referenzelektrode 20 so um das Isolationsprofil 32 gruppiert, dass die mindestens eine Arbeitselektrode 16, die mindestens eine Gegenelektrode 18 sowie die mindestens eine Referenzelektrode 20 voneinander isoliert sind. Die Zusammenführungsstation 130 verlässt eine Elektrodenanordnung, die mindestens eine beschichtete Arbeitselektrode 16, mindestens eine, bevorzugt unbeschichtet ausgeführte Gegenelektrode 18, mindestens eine Referenzelektrode 20 sowie das Isolationsprofil 32 umfasst. Die die Zusammenführungsstation 130 verlassende Elektrodenanordnung 16, 18, 20 stellt ein Elektrodenpaket 132 dar.

Der Darstellung gemäß Figur 5 ist ein weiterer Herstellungsschritt, dem das Elektrodenpaket gemäß Figur 4 nach der Zusammenführung zugeführt wird, zu entnehmen. Figur 5 zeigt, dass das Elektrodenpaket 132, welches die mindestens eine beschichtete Arbeitselektrode 110, die mindestens eine bevorzugt unbeschichtete Gegenelektrode 18 sowie die mindestens eine Referenzelektrode 20 umfasst, einer Immobilisierungsmedium-Beschichtung 140 zugeführt wird. Im Rahmen der in Figur 5 dargestellten Immobilisierungsmedium-Beschichtung 140 wird zur Immobilisierung reaktiver Bestandteile zum Beispiel ein Immobilisierungsmedium 142 auf das gemäß der Darstellung in Figur 5 in eine Ringdüse 146 einlaufende Elektrodenpaket 132 aufgebracht. Hierdurch wird das Elektrodenpaket zusätzlich isoliert und/oder giftige Bestandteile (beispielsweise das als Zellgift wirkende GOD) von einer Diffusion in das Körpergewebe abgehalten. Die Außenseite des elektrochemischen Sensors 10, welche mit dem Körpergewebe in Berührung kommt, sollte biokompatibel sein (d.h. vom Körper nicht abgestoßen werden). Das ist eine weitere besonders bevorzugte Eigenschaft des Immobilisierungsmediums 142. Alternativ oder zusätzlich kann auch eine zusätzliche Schicht aufgebracht werden, welche diese zusätzliche Eigenschaft der Biokompatibilität gewährleistet. Auch die weiteren eingesetzten Materialien, welche mit dem Körpergewebe in Berührung kommen, beispielsweise Materialien zur Isolation im Steckerbereich und am Einführungsende, sollten entsprechende biokompatible Eigenschaften besitzen oder entsprechend beschichtet sein.

Die im Rahmen der Immobilisierungsmedium-Beschichtung 140 eingesetzte Ringdüse 146 umfasst einen Hohlraum 150, der eine Austrittsöffnung 148 aufweist und mit einem Immobilisierungsmedium 142 befüllt ist. Der Hohlraum 150 der Ringdüse 146 wird kontinuierlich mit dem Immobilisierungsmedium 142 befüllt, so dass die gesamte Oberfläche des in den Hohlraum in Förderrichtung 80 einlaufenden Elektrodenpakets 132 durch das Immobilisierungsmedium 142 benetzt ist. An der Austrittsöffnung 148 der Ringdüse 146 ist die Mantelfläche des Elektrodenpakets 132, welches in Förderrichtung 80 in die Ringdüse 146 einläuft, mit einer Beschichtung mit Immobilisierungsmedium 142 versehen. Die Förderrichtung des Elektrodenpakets 132 ist in der Darstellung gemäß Figur 5 mit Bezugszeichen 144 bezeichnet. Bezugszeichen 152 bezeichnet das mit dem Immobilisierungsmedium 142 beschichtete Elektrodenpaket 132.

Anstelle des in Figur 5 in die Ringdüse 146 in Förderrichtung 144 eintretenden Elektrodenpakets 132, welches die mindestens eine Arbeitselektrode 16, die mindestens eine Gegenelektrode 18 sowie die mindestens eine Referenzelektrode 20 umfasst, welche durch das Isolationsprofil 32 gegeneinander isoliert sind, können auch einzelne der mindestens einen Arbeitselektrode 16, der mindestens einen Gegenelektrode 18 sowie der mindestens einen Referenzelektrode 20 der Immobilisierungsmedium-Beschichtung 140 zugeführt werden. Über die Immobilisierungsmedium-Beschichtung 140, die gemäß Figur 5 aufgetragen werden kann, erfolgt der Kontakt zwischen der mindestens einen beschichteten Arbeitselektrode 110 und der mindestens einen, bevorzugt unbeschichtet ausgebildeten Gegenelektrode 18 und dem Körpergewebe oder der Körperflüssigkeit, wodurch das Vorhandensein eines bestimmten Analyten zu bestimmen ist. Der Darstellung gemäß Figur 6 ist ein Konfektionierungsschritt entnehmbar.

Gemäß der in Figur 6 schematisch dargestellten Konfektionierung 160 wird das gemäß der vorstehenden Herstellungsschritte bereitgestellte, beschichtete Elektrodenpaket 152, welches am Umfang zum Beispiel mit dem Immobilisierungsmedium 142 beschichtet ist, in einzelne Sektionen 164 konfektioniert. Die Konfektionierung 160 erfolgt bevorzugt durch ein Querschneiden des beschichteten Elektrodenpakets 152. Dabei können unterschiedliche Längen 162 der konfektionierten Sektionen 164 je nach Anwendungszweck eingestellt werden.

An die gemäß Konfektionierung 160 hergestellten Sektionen 164 wird an einem Ende der in Figur 2 dargestellte Einführkopf 60 samt erster Abdichtung 62 befestigt und am anderen Ende der in Figur 2 dargestellte Anschlussträger 64, an dem die Anschlüsse 22, 24, 26 entsprechend der Anzahl der Elektroden des Elektrodenpakets 132 angeschlossen werden, befestigt. So wird die in Figur 2 nicht in kompletter Länge dargestellte Vorrichtung, die einen elektrochemischen Sensor 10 umfasst, erhalten. Die elektrochemische Messzelle des elektrochemischen Sensors 10 wird durch die Mantelfläche der im Rahmen der Immobilisierungsmedium-Beschichtung 140 aufgebrachten Schicht des Immobilisierungsmediums 142 ausgebildet. Diese Schicht aus Immobilisierungsmedium 142 stellt die Begrenzung der elektrochemischen Messzelle und die Kontaktfläche dar, mit welcher der elektrochemische Sensor 10 mit dem Körpergewebe bzw. der Körperflüssigkeit in Kontakt steht.

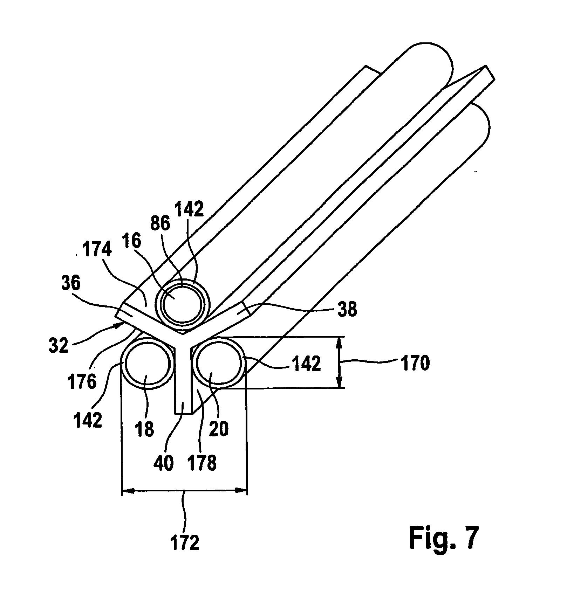

Der Darstellung gemäß Figur 7 ist eine perspektivische Ansicht der Elektrodenanordnung des elektrochemischen Sensors 10 zu entnehmen.

Aus der Darstellung gemäß Figur 7 geht hervor, dass die mindestens eine Arbeitselektrode 16, die mindestens eine Gegenelektrode 18 sowie die mindestens eine Referenzelektrode 20 über das Isolationsprofil 32 voneinander getrennt sind. Das in der Darstellung gemäß Figur 7 eine Y-Geometrie 34 aufweisende Isolationsprofil 32 umfasst den ersten Steg 36, den zweiten Steg 38 sowie den dritten Steg 40. Der erste Steg 36 und der zweite Steg 38 begrenzen ein erstes Aufnahmefach 174, in welchem die mindestens eine Arbeitselektrode 16 aufgenommen ist. Die mindestens eine Arbeitselektrode 16 weist eine Beschichtung mit einem Reagenzmedium 86 eines Reagenz zum Nachweis des Analyten im Körpergewebe oder in der Körperflüssigkeit auf. Aus der Darstellung gemäß Figur 7 geht hervor, dass auf dieses Reagenzmedium 86 das Immobilisierungsmedium 142 aufgebracht sein kann. In der in Figur 7 dargestellten Ausführungsvariante der Elektrodenanordnung 16, 18, 20 des Elektrodenpakets 132 ist das Immobilisierungsmedium 142 auf die Mantelfläche der mindestens einen Arbeitselektrode 16, der mindestens einen Gegenelektrode 18 sowie der mindestens einen Referenzelektrode 20 aufgebracht. Wie vorstehend im Zusammenhang mit Figur 5 beschrieben, kann auch das gesamte aus der Zusammenführungsstation 130 gemäß Figur 4 austretende Elektrodenpaket 132 als Ganzes mit dem Immobilisierungsmedium 142 beschichtet werden, welches dann das in Figur 1 dargestellte Einbettungsmaterial 14 darstellt.

Der perspektivischen Darstellung gemäß Figur 7 ist des Weiteren zu entnehmen, dass der zweite Steg 38 mit dem dritten Steg 40 des Isolationsprofils 42 ein drittes Aufnahmefach 178 begrenzt, in welchem im Ausführungsbeispiel gemäß Figur 7 die mindestens eine Referenzelektrode 20 aufgenommen ist. Schließlich begrenzen der dritte Steg 40 und der erste Steg 36 des Isolationsprofils 32 gemäß der Darstellung in Figur 7 ein zweites Aufnahmefach 176, in welchem sich die mindestens eine Gegenelektrode 18 der Elektrodenanordnung 16, 18, 20 befindet. Aus der Darstellung gemäß Figur 7 geht hervor, dass ein Durchmesser 170 der Referenzelektrode in der Größenordnung von etwas 100 µm liegt, während mit Bezugszeichen 172 die Summe der Durchmesser der mindestens einen Referenzelektrode 20, der mindestens einen Gegenelektrode 18 sowie der Materialdicke des dritten Stegs 40 des Isolationsprofils 32 bezeichnet ist. Die Strecke 172 gemäß der Darstellung in Figur 7 weist eine Länge in der Größenordnung von etwas 250 µm auf.

Aus den genannten Abmessungen der Elektrodenanordnung 16, 18, 20 gemäß der perspektivischen Darstellung in Figur 7 geht hervor, dass die vorgeschlagene Elektrodenanordnung 16, 18, 20 des erfindungsgemäß vorgeschlagenen elektrochemischen Sensors 10 sehr kompakte Abmessungen aufweist, was auf der parallelen Anordnung der mindestens einen Arbeitselektrode 16, der mindestens einen Gegenelektrode 18 und der mindestens einen Referenzelektrode 20 in den Aufnahmefächern 174, 176 und 178 des Isolationsprofils 32 beruht.

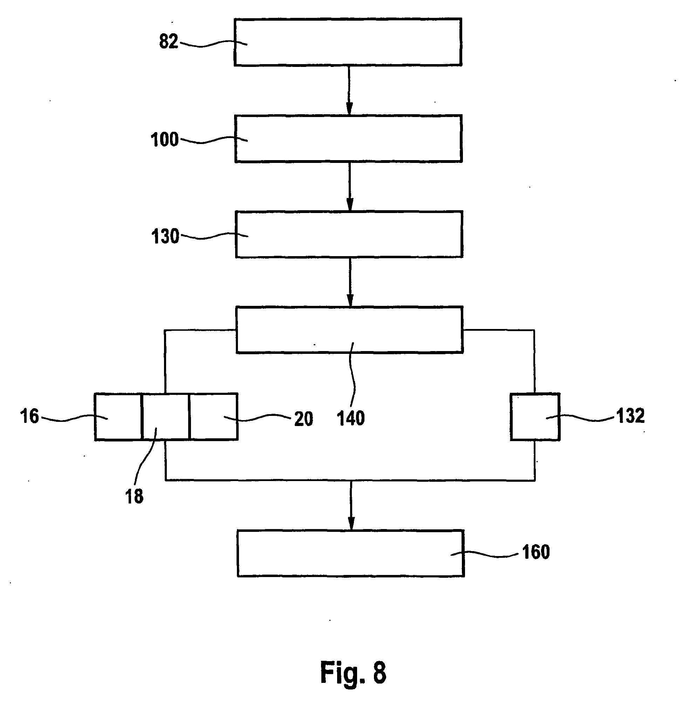

Der Darstellung gemäß Figur 8 ist eine schematische Darstellung des Herstellungsverfahrens zur Herstellung des elektrochemischen Sensors, insbesondere der Elektrodenanordnung zu entnehmen.

Aus dem Fließbild gemäß Figur 8 geht hervor, dass im Rahmen der Ringdüsenbeschichtung 82 die mindestens eine Arbeitselektrode 16 mit dem Reagenzmedium 86 beschichtet wird, welches ein zum Nachweis des Analyten geeignetes Reagenz darstellt. Im sich anschließenden Trocknungsschritt innerhalb der Trocknungsstation 100 wird das Reagenzmedium 86, welches sich nach der Ringdüsenbeschichtung 82 an der Oberfläche 94 der mindestens einen Arbeitselektrode 16 befindet, getrocknet. Anschließend erfolgt die Zusammenführung der nunmehr mit dem Reagenzmedium 86 an der Oberfläche 94 beschichteten, mindestens einen Arbeitselektrode 16 mit der bevorzugt unbeschichtet ausgebildeten, mindestens einen Gegenelektrode 18, der mindestens einen Referenzelektrode 20 und dem bevorzugt im Wege des Strangextrusionsverfahrens hergestellten Isolationsprofils 32. Die Zusammenführung erfolgt in einer Zusammenführungsstation 130. Das aus der Zusammenführungsstation 130 erhaltene Elektrodenpaket 132 wird nachfolgend einer Immobilisierungsmedium-Beschichtung 140 unterzogen. Im Rahmen der Immobilisierungsmedium-Beschichtung 140 ist es möglich, das Immobilisierungsmedium 142 sowohl auf das in der Zusammenführungsstation 130 erhaltene Elektrodenpaket 132 als Ganzes aufzubringen und dieses demzufolge im Immobilisierungsmedium einzubetten. Daneben besteht die Möglichkeit, auch die mindestens eine Arbeitselektrode 16, die mindestens eine Gegenelektrode 18 sowie die mindestens eine Referenzelektrode 20 separat zu beschichten und diese beschichteten Einzelelektroden in der Zusammenführungsstation 130 zusammenzuführen.

Im Rahmen der sich an die Immobilisierungsmedium-Beschichtung 140 anschließenden Konfektionierung 160 erfolgt eine Trennung entweder des beschichteten Elektrodenpakets 152 oder der zusammengeführten einzeln beschichteten Einzelelektroden 16, 18 und 20. Im Rahmen der Konfektionierung 160 werden Sektionen 164 hergestellt, die in unterschiedlichen Längen 162 ausgebildet sein können, wobei sich die Länge 162 je nach der Anwendung der Elektrodenanordnung zum Einsatz in einem elektrochemischen Sensor 10 richtet.

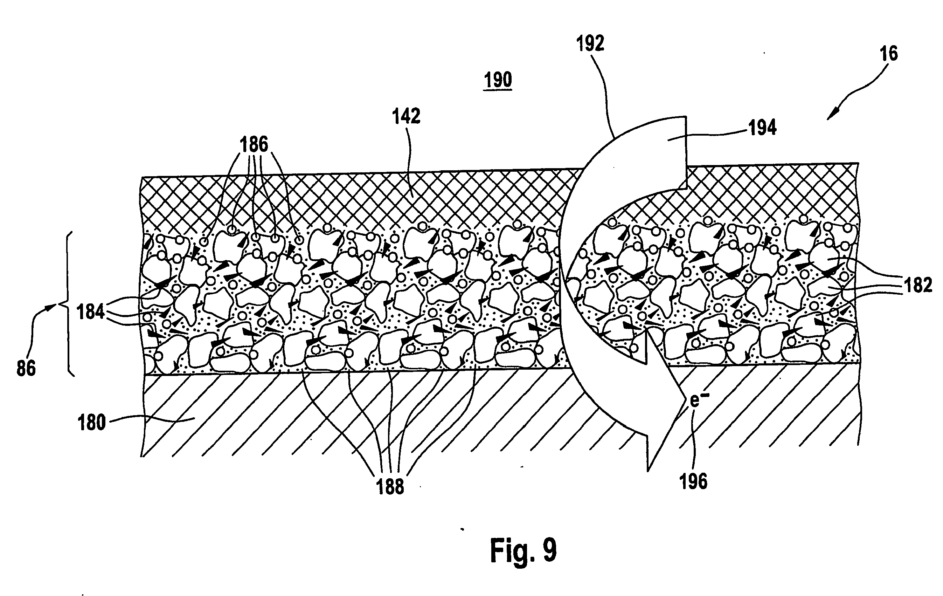

In Figur 9 ist schematisch ein Ausschnitt einer Elektrodenoberfläche einer Arbeitselektrode 16 in Schnittdarstellung gezeigt. Die Arbeitselektrode weist in diesem Beispiel einen Golddraht 180 auf. Der Golddraht 180 ist gemäß dem in Figur 8 dargestellten Verfahren mit einem Reagenzmedium 86 beschichtet. Das Reagenzmedium setzt sich in diesem Beispiel aus drei verschiedenen Komponenten zusammen: leitfähigen Kohlenstoff-Partikeln 182, Braunstein-Partikeln 184, GOD-Partikeln bzw. GOD-Konglomeraten 186 sowie einem Binder-Polymer 188. Das Binder-Polymer 188 gewährleistet die Verarbeitungseigenschaften des Reagenzmediums im ungetrockneten Zustand. Dabei wird das Reagenzmedium 86 durch Wahl des Binder-Polymers 188 in seiner Viskosität und/oder Oberflächenspannung so eingestellt, dass sich dieses im "nassen" (d. h. ungetrockneten) Zustand durch Ringdüsenbeschichtung 82 gut verarbeiten lässt und eine homogene, gleichmäßige, gut auf dem Golddraht 180 haftende Schicht bildet. Gleichzeitig wird das Binder-Polymer derart gewählt, dass sich dieses bei moderaten Temperaturen trocknen lässt, ohne dass beispielsweise GOD 186 bei diesem Trocknungsschritt thermisch zerstört wird. Als geeignetes Binder-Polymer 188 bieten sich beispielsweise sowie auch Gemische, beispielsweise Gemische aus Polymeren mit verschiedenen Lösungsmitteln, an.

Weiterhin ist in Figur 9 auch die Schicht des Immobilisierungsmediums 142 dargestellt. Diese umgibt das Reagenzmedium 86 und verhindert, dass Körperflüssigkeit (hier symbolisch mit 190 bezeichnet) in unmittelbaren Kontakt mit den Braunstein-Partikeln 184 und mit den GOD-Partikeln 186 gerät und dass GOD in die Körperflüssigkeit 190 eindiffundieren kann. Gleichzeitig kann Sauerstoff und Glucose als nachzuweisender Analyt aus der Körperflüssigkeit 190 durch die Schicht des Immobilisierungsmediums 142 diffundieren und so zu dem Reagenzmedium 86 gelangen.

Schließlich ist in Figur 9 symbolisch die zum Nachweis von Glucose 194 in Körperflüssigkeit 190 eingesetzte Reaktion durch einen "Reaktionspfeil" 192 dargestellt. Die Glucose 194 wird über das Enzym GOD 186 zu Gluconolacton oxidiert, und anschließend wird durch das Enzym GOD 186 Sauerstoff zu H₂O₂ reduziert. Anschließend wird dann das H₂O₂ katalytisch vom Braunstein 184 oxidiert und dabei die Elektronen über die mit dem Braunstein 184 in Kontakt stehenden Kohlenstoff-Partikel 182 an den ableitenden Golddraht 180 übertragen. Das so beeinflusste Potential des Golddrahtes 180 (bzw. der gesamten Arbeitselektrode 16) kann auf die oben beschriebene Weise, beispielsweise durch eine amperometrische Messung, erfasst und daraus auf die Konzentration der Glucose in der Körperflüssigkeit 190 geschlossen werden.

## Patentansprüche

1. Elektrochemischer Sensor (10) zur Ermittlung einer Konzentration mindestens eines Analyten in einem Medium, insbesondere einem Körpergewebe und/oder einer Körperflüssigkeit, wobei der elektrochemische Sensor (10) ein Isolationselement (32) und mindestens zwei Elektroden (16, 18, 20) aufweist, wobei die mindestens zwei Elektroden (16, 18, 20) mindestens eine Arbeitselektrode (16) und mindestens eine weitere Elektrode (18, 20), insbesondere mindestens eine Gegenelektrode (18) und/oder mindestens eine Referenzelektrode (20), umfassen, wobei die mindestens zwei Elektroden (16, 18, 20) zumindest teilweise als im Wesentlichen parallel zueinander verlaufende drahtförmige Elektroden mit umfangsseitigen Elektrodenflächen ausgebildet sind, wobei sich die mindestens zwei Elektroden (16, 18, 20) entlang eines profilförmig ausgeführten Isolationselements (32) erstrecken, **dadurch gekennzeichnet, dass** das profilförmig ausgeführt Isolationselement (32) mindestens zwei Aufnahmefächer (174, 176, 178) für die mindestens zwei Elektroden (16, 18, 20) aufweist.

2. Elektrochemischer Sensor gemäß Anspruch 1, **dadurch gekennzeichnet, dass** das profilförmig ausgeführte Isolationselement (32) einen Y-förmigen, einen kreuzförmigen oder einen sternförmigen Querschnitt (34) aufweist.

3. Elektrochemischer Sensor gemäß einem der vorhergehenden Ansprüche, **dadurch gekennzeichnet, dass** die mindestens zwei Elektroden (16, 18, 20) entweder einzeln oder in Form eines Elektrodenpakets (132) ganz oder teilweise von mindestens einer Schicht eines Immobilisierungsmediums (142) ummantelt sind, wobei das Immobilisierungsmedium (142) eine zumindest teilweise Permeabilität für den mindestens einen Analyten aufweist, wobei das Immobilisierungsmedium (142) vorzugsweise ein Polyurethan und/oder ein weiteres Polymermaterial aufweist.

4. Elektrochemischer Sensor gemäß einem der vorhergehenden Ansprüche, **dadurch gekennzeichnet, dass** mindestens eine Gegenelektrode (18) vorgesehen ist, wobei die mindestens eine Gegenelektrode (18) bevorzugt aus einem für elektrochemische Messungen geeignetem Material aus der Gruppe umfassend Gold, Silber, Palladium, Platin oder Kohlenstoff gefertigt ist.

5. Elektrochemischer Sensor gemäß einem der vorhergehenden Ansprüche, **dadurch gekennzeichnet, dass** mindestens eine Referenzelektrode vorgesehen ist, wobei die mindestens eine Referenzelektrode (20) bevorzugt Ag aufweist und an der Oberfläche mit AgCl belegt und zumindest teilweise, vorzugsweise vollständig, vom einem Immobilisierungsmedium (142) ummantelt ist.

6. Vorrichtung (10, 60, 64) zur Ermittlung einer Konzentration mindestens eines Analyten in einem Medium, insbesondere einem Körpergewebe und/oder einer Körperflüssigkeit, mit mindestens einem elektrochemischen Sensor (10) gemäß einem der vorhergehenden Ansprüche, mit mindestens einer Spannungsmessvorrichtung und/oder mindestens einer Strommessvorrichtung zur Messung einer Spannung und/oder eines Stromes zwischen der mindestens einen Arbeitselektrode (16) und der mindestens einen Gegenelektrode (18) und/oder der mindestens einen Referenzelektrode, sowie vorzugsweise zusätzlich mit einem Einführkopf (60) und/oder einer Einführhilfe.

7. Vorrichtung (10, 60, 64) gemäß dem vorhergehenden Anspruch, **dadurch gekennzeichnet, dass** der elektrochemische Sensor (10) mit einem elektrischen Anschluss (64), vorzugsweise einem als Formschlussteil ausgebildeten elektrischen Anschluss (64), verbunden ist, wobei der elektrische Anschluss (64) vorzugsweise mindestens zwei elektrische Anschlüsse (22, 24, 26) für die mindestens zwei Elektroden (16, 18, 20) zur elektrischen Kontaktierung von deren Oberflächen aufweist.

8. Vorrichtung (10, 60, 64) gemäß dem vorhergehenden Anspruch, **dadurch gekennzeichnet, dass** der elektrische Anschluss eine Schneidklemmverbindung und/oder eine Insertionshilfe und/oder eine Hohlnadel aufweist.

9. Verwendung einer Vorrichtung (10, 60, 64) gemäß einem der vorhergehenden, eine Vorrichtung (10, 60, 64) betreffenden Ansprüche für eine kontinuierliche Ermittlung einer Konzentration mindestens eines Analyten in einem Medium, insbesondere einem Körpergewebe und/oder einer Körperflüssigkeit.

10. Verfahren zur Herstellung eines elektrochemischen Sensors (10) zur Ermittlung einer Analytkonzentration in einem Medium, insbesondere einem Körpergewebe und/oder einer Körperflüssigkeit, mit nachfolgenden Verfahrensschritten:
a) mindestens eine der mindestens zwei Elektroden (16, 18, 20) wird mit einem Reagenzmedium (86) zum Nachweis mindestens eines Analyten beschichtet,
b) die mindestens zwei Elektroden (16, 18, 20) und ein profilförmig ausgeführtes Isolationsprofil (32) werden nach Durchführung des Verfahrensschrittes a) in einer Zusammenführungsstation (130) zusammengeführt, so dass sich die mindestens zwei Elektroden (16, 18, 20) entlang des profilförmig ausgeführten Isolationselements (32) erstrecken, wobei das profilförmig ausgeführte Isolationselement (32) mindestens zwei Aufnahmefächer (174, 176, 178) für die mindestens zwei Elektroden (16, 18, 20) aufweist, so dass nach der Zusammenführungsstation (130) ein strangförmiges Elektrodenpaket (132) erhalten wird,
c) einzelne der mindestens zwei Elektroden (16, 18, 20) oder das erhaltende Elektrodenpaket (132) werden einer Immobilisierungsmedium-Beschichtung (140) unterzogen und
d) ein erhaltenes, mit einer Immobilisierungsmedium-Beschichtung (140) versehenes Elektrodenpaket (132) wird einer Konfektionierung (160) zur Vereinzelung zugeführt.

11. Verfahren nach dem vorhergehenden Anspruch, **dadurch gekennzeichnet, dass** nach Verfahrensschritt a) die mindestens eine der beiden Elektroden (16, 18, 20) eine Trocknungsstation (100) passiert.

12. Verfahren gemäß Anspruch 10, **dadurch gekennzeichnet, dass** die Verfahrensschritte a) und/oder c) mittels eines Ringdüsen-Beschichtungsverfahrens (82) und/oder eines galvanischen Verfahrens durchgeführt werden.

13. Verfahren gemäß dem vorhergehenden Anspruch, **dadurch gekennzeichnet, dass** die Beschichtung der mindestens einen der mindestens zwei Elektroden (16, 18, 20) mit dem Reagenzmedium (86) gemäß Verfahrensschritt a) in einer ersten Ringdüse (84), die Oberfläche (94) der mindestens einen der mindestens zwei Elektroden (16, 18, 20) bevorzugt vollständig benetzend, erfolgt.

14. Verfahren gemäß Anspruch 12, **dadurch gekennzeichnet, dass** die Beschichtung gemäß Verfahrensschritt c) in einer zweiten Ringdüse (146) erfolgt, in der entweder das gemäß Verfahrensschritt b) erhaltene Elektrodenpaket (132) bevorzugt voll umfänglich mit einem Immobilisierungsmedium (142) oder einzelne der mindestens zwei Elektroden (16, 18, 20) bevorzugt voll umfänglich mit einem Immobilisierungsmedium (142) beschichtet werden.

15. Verfahren gemäß Anspruch 10, **dadurch gekennzeichnet, dass** gemäß Verfahrensschritt d) die Konfektionierung (160) des mit einer Immobilisierungsmedium-Beschichtung (140), bevorzugt eines Immobilisierungsmediums (142), beschichteten Elektrodenpakets (132) durch Abtrennen einzelner Sektionen (164) in anwendungsspezifischen Längen (162) erfolgt.

## Claims

1. Electrochemical sensor (10) for the determination of a concentration of at least one analyte in a medium, in particular a body tissue and/or a body fluid, the electrochemical sensor (10) having an isolation element (32) and at least two electrodes (16, 18, 20), the at least two electrodes (16, 18, 20) comprising at least one working electrode (16) and at least one further electrode (18, 20), in particular at least one counterelectrode (18) and/or at least one reference electrode (20), the at least two electrodes (16, 18, 20) being at least partially formed as wire-shaped electrodes running substantially parallel to one another and with circumferential electrode surfaces, the at least two electrodes (16, 18, 20) extending along an isolation element (32) designed with a profiled shape, **characterized in that** the isolation element (32) designed with a profiled shape has at least two holding compartments (174, 176, 178) for the at least two electrodes (16, 18, 20).

2. Electrochemical sensor according to Claim 1, **characterized in that** the isolation element (32) designed with a profiled shape has a Y-shaped, a cross-shaped or a star-shaped cross section (34).

3. Electrochemical sensor according to one of the preceding claims, **characterized in that** the at least two electrodes (16, 18, 20) are enveloped wholly or partly by at least one layer of an immobilization medium (142), either individually or in the form of an electrode pack (132), the immobilization medium (142) being at least partly permeable to the at least one analyte, the immobilization medium (142) preferably having a polyurethane and/or a further polymer material.

4. Electrochemical sensor according to one of the preceding claims, **characterized in that** at least one counterelectrode (18) is provided, the at least one counterelectrode (18) preferably being produced from a material, suitable for electrochemical measurements, from the group comprising gold, silver, palladium, platinum or carbon.

5. Electrochemical sensor according to one of the preceding claims, **characterized in that** at least one reference electrode is provided, the at least one reference electrode (20) preferably exhibiting Ag and being coated on the surface with AgCl, and being enveloped at least partly, preferably completely, by an immobilization medium (142).

6. Apparatus (10, 60, 64) for the determination of a concentration of at least one analyte in a medium, in particular a body tissue and/or a body fluid, having at least one electrochemical sensor (10) according to one of the preceding claims, having at least one voltage measuring device and/or at least one current measuring device for measuring a voltage and/or a current between the at least one working electrode (16) and the at least one counterelectrode (18) and/or the at least one reference electrode, as well as preferably additionally having an insertion head (60) and/or an insertion aid.

7. Apparatus (10, 60, 64) according to the preceding claim, **characterized in that** the electrochemical sensor (10) is connected to an electrical connection (64), preferably an electrical connection (64) formed as a positive locking part, the electrical connection (64) preferably having at least two electrical connections (22, 24, 26) for the at least two electrodes (16, 18, 20) for the electrical contact with their surfaces.

8. Apparatus (10, 60, 64) according to the preceding claim, **characterized in that** the electrical connection has an insulation displacement connection and/or an insertion aid and/or a hollow needle.

9. Use of an apparatus (10, 60, 64) according to one of the preceding claims, which relate to an apparatus (10, 60, 64), for a continuous determination of a concentration of at least one analyte in a medium, in particular a body tissue and/or a body fluid.

10. Method for producing an electrochemical sensor (10) for the determination of an analyte concentration in a medium, in particular a body tissue and/or a body fluid, having the following method steps:
a) at least one of the at least two electrodes (16, 18, 20) is coated with a reagent medium (86) for detecting at least one analyte,
b) after the method step a) has been carried out, the at least two electrodes (16, 18, 20) and an isolation profile (32) designed with a profiled shape are assembled in an assembly station (130), so that the at least two electrodes (16, 18, 20) extend along the isolation element (32) designed with a profiled shape, the isolation element (32) designed with a profiled shape having at least two holding compartments (174, 176, 178) for the at least two electrodes (16, 18, 20), so that after the assembly station (130) a slab-shaped electrode pack (132) is obtained,
c) individual ones of the at least two electrodes (16, 18, 20) or the electrode pack (132) obtained are subjected to an immobilization medium coating operation (140), and
d) an obtained electrode pack (132) provided with a coating (140) of immobilization medium is fed to a cut-to-length operation (160) for separation.

11. Method according to the preceding claim, **characterized in that** after method step a) the at least one of the two electrodes (16, 18, 20) passes through a drying station (100).

12. Method according to Claim 10, **characterized in that** the method steps a) and/or c) are carried out by means of an annular nozzle coating method (82) and/or an electroplating method.

13. Method according to the preceding claim, **characterized in that** the coating of the at least one of the at least two electrodes (16, 18, 20) with the reagent medium (86) in accordance with method step a) is performed in a first annular nozzle (84) in a fashion preferably completely wetting the surface (94) of the at least one of the at least two electrodes (16, 18, 20).

14. Method according to Claim 12, **characterized in that** the coating in accordance with method step c) is performed in a second annular nozzle (146) in which either the electrode pack (132) obtained in accordance with method step b) is preferably coated to the full extent with an immobilization medium (142), or individual ones of the at least two electrodes (16, 18, 20) are preferably coated to the full extent with an immobilization medium (142).

15. Method according to Claim 10, **characterized in that** in accordance with method step d) the cut-to-length operation (160) of the electrode pack (132) coated with a coating (140) of immobilization medium, preferably of an immobilization medium (142), is performed by separating individual sections (164) in application-specific lengths (162).

## Revendications

1. Détecteur électrochimique (10) destiné à déterminer la concentration d'au moins un analyte dans un fluide, en particulier dans un tissu corporel et/ou un liquide corporel,
le détecteur électrochimique (10) présentant un élément d'isolation (32) et au moins deux électrodes (16, 18, 20),
les deux ou plusieurs électrodes (16, 18, 20) comprenant au moins une électrode de travail (16) et au moins une autre électrode (18, 20), en particulier au moins une contre-électrode (18) et/ou au moins une électrode de référence (20),
les deux ou plusieurs électrodes (16, 18, 20) étant formées au moins en partie comme électrodes en forme de fils s'étendant essentiellement en parallèle l'un à l'autre et dotés de surfaces périphériques d'électrodes,
les deux ou plusieurs électrodes (16, 18, 20) s'étendant le long d'un élément d'isolation (32) de forme profilée,
**caractérisé en ce que**
l'élément d'isolation (32) de forme profilée présente au moins deux compartiments de réception (174, 176, 178) pour les deux ou plusieurs électrodes (16, 18, 20).

2. Détecteur électrochimique selon la revendication 1, **caractérisé en ce que** l'élément d'isolation (32) de forme profilée a une section transversale (34) en forme de Y, en forme de croix ou en forme d'étoile.

3. Détecteur électrochimique selon l'une des revendications précédentes, **caractérisé en ce que** les deux ou plusieurs électrodes (16, 18, 20) sont entourées totalement ou partiellement, séparément ou sous la forme d'un paquet d'électrodes (132), par au moins une couche d'un fluide d'immobilisation (142), le fluide d'immobilisation (142) présentant une perméabilité au moins partielle vis-à-vis du ou des analytes, le fluide d'immobilisation (142) présentant de préférence un polyuréthane et/ou un autre matériau polymère.

4. Détecteur électrochimique selon l'une des revendications précédentes, **caractérisé en ce qu'**il présente au moins une contre-électrode (18), la ou les contre-électrodes (18) étant réalisées de préférence en un matériau qui convient pour des mesures électrochimiques et sélectionné dans l'ensemble constitué de l'or, de l'argent, du palladium, du platine ou du carbone.

5. Détecteur électrochimique selon l'une des revendications précédentes, **caractérisé en ce qu'**il présente au moins une électrode de référence, la ou les électrodes de référence (20) présentant de préférence de l'Ag, leur surface étant occupée par de l'AgCl et étant entourée au moins en partie et de préférence complètement par un fluide d'immobilisation (142).

6. Dispositif (10, 60, 64) de détermination de la concentration d'au moins un analyte dans un fluide, en particulier dans un tissu corporel et/ou un liquide corporel, et présentant au moins un détecteur électrochimique (10) selon l'une des revendications précédentes et au moins un dispositif de mesure de tension et/ou au moins un dispositif de mesure de courant qui mesure la tension et/ou le courant entre la ou les électrodes de travail (16), la ou les contre-électrodes (18) et/ou la ou les électrodes de référence, ainsi que de préférence une tête d'insertion (60) et/ou un accessoire d'insertion.

7. Dispositif (10, 60, 64) selon la revendication précédente, **caractérisé en ce que** le détecteur électrochimique (10) est relié à un raccordement électrique (64), de préférence un raccordement électrique (64) configuré comme pièce de raccordement en correspondance géométrique, le raccordement électrique (64) présentant de préférence au moins deux raccordements électriques (22, 24, 26) qui assurent le contact électrique avec la surface des deux ou plusieurs électrodes (16, 18, 20).

8. Dispositif (10, 60, 64) selon la revendication précédente, **caractérisé en ce que** le raccordement électrique présente une liaison à pince coupante et/ou un accessoire d'insertion et/ou une aiguille creuse.

9. Utilisation d'un dispositif (10, 60, 64) selon l'une des revendications précédentes concernant le dispositif (10, 60, 64), en vue de la détermination en continu de la concentration d'au moins un analyte dans un fluide, en particulier dans un tissu corporel et/ou un liquide corporel.

10. Procédé de fabrication d'un détecteur électrochimique (10) destiné à déterminer la concentration d'un analyte dans un fluide, en particulier dans un tissu corporel et/ou un liquide corporel, et présentant les étapes de traitement suivantes :
a) au moins l'une des deux ou plusieurs électrodes (16, 18, 20) est recouverte par un fluide réactif (86) en vue de la détection d'au moins un analyte,
b) les deux ou plusieurs électrodes (16, 18, 20) et un profilé d'isolation (32) sont rassemblés dans un poste de rassemblement (130) après l'exécution de l'étape de traitement a) de telle sorte que les deux ou plusieurs électrodes (16, 18, 20) s'étendent le long de l'élément d'isolation (32) de forme profilée, l'élément d'isolation (32) de forme profilée présentant au moins deux compartiments de réception (174, 176, 178) pour les deux ou plusieurs électrodes (16, 18, 20) de telle sorte que l'on obtienne après le poste (130) de rassemblement un paquet d'électrodes (132) en forme de barreau,
c) un revêtement (140) par un fluide d'immobilisation est appliqué sur certaines des deux ou plusieurs électrodes (16, 18, 20) ou sur le paquet d'électrodes (132) obtenu et
d) le paquet d'électrodes (132) ainsi obtenu et doté d'un revêtement (140) par un fluide d'immobilisation est amené à un conditionnement (160) pour être séparé des autres.

11. Procédé selon la revendication précédente, **caractérisé en ce qu'**après l'étape de traitement a), au moins l'une des deux électrodes (16, 18, 20) passe par un poste de séchage (100).

12. Procédé selon la revendication 10, **caractérisé en ce que** les étapes de traitement a) et/ou c) sont exécutées au moyen d'un procédé (82) de revêtement par tuyère annulaire et/ou par un procédé galvanique.

13. Procédé selon la revendication précédente, **caractérisé en ce que** le revêtement de la ou des deux électrodes (16, 18, 20) par le fluide réactif (86) lors de l'étape de traitement a) s'effectue dans une première tuyère annulaire (84), en mouillant de préférence complètement la surface (94) de la ou des deux ou plusieurs électrodes (16, 18, 20).

14. Procédé selon la revendication 12, **caractérisé en ce que** le revêtement de l'étape de traitement c) s'effectue dans une deuxième tuyère annulaire (146) dans laquelle le paquet d'électrodes (132) obtenu dans l'étape de traitement b) est revêtu de préférence sur toute sa périphérie par un fluide d'immobilisation (142) ou certaines des deux ou plusieurs électrodes (16, 18, 20) sont revêtues de préférence sur toute leur périphérie par un fluide d'immobilisation (142).

15. Procédé selon la revendication 10, **caractérisé en ce que** dans l'étape de traitement d), le conditionnement (160) du paquet d'électrodes (132) revêtu par un revêtement (140) de fluide d'immobilisation et de préférence du fluide d'immobilisation (142) s'effectue en découpant des segments (164) qui présentent des longueurs (162) spécifiques à l'application.
